# EUROPEAN PATENT SPECIFICATION

(11) **EP 1 994 904 B1**
(45) Date of publication and mention of the grant of the patent: **10.05.2017**
(21) Application number: 08009510.2
(22) Date of filing: 23.05.2008
(51) Int. Cl.: A61B 18/00, A61B 18/14

(54) **High frequency surgical instrument**
Chirurgisches Hochfrequenzinstrument
Instrument chirurgical à hautes fréquences

(30) Priority: 25.05.2007 JP 2007138823
(43) Date of publication of application: 26.11.2008
(73) Proprietor: FUJIFILM Corporation, Tokyo (JP); Akahoshi, Kazuya, Kasuya-gun Fukuoka (JP)
(72) Inventor: Akahane, Hidefumi, Kita-ku, Saitama-shi, Saitama (JP); Akahoshi, Kazuya, Fukuoka (JP)
(74) Representative: Höhfeld, Jochen

(56) References cited:
- EP-A- 0 546 767
- EP-A- 0 712 608
- WO-A1-97/49345
- US-A- 5 330 471
- US-A- 5 620 459
- US-A1- 2002 111 624
- US-A1- 2006 264 930

## Description

### [TECHNICAL FIELD]

This invention relates to an endoscopic high frequency surgical instrument having a forceps manipulably attached on a fore distal end of an elongated insertion member.

### [DISCLOSURE OF THE INVENTION]

### [BACKGROUND OF THE INVENTION]

Generally, a high frequency surgical instrument to be introduced into a patient's body cavity for surgical or biopsy purposes has a high frequency electrode mounted on a fore distal end of an elongated insertion member. Electric current is conducted through the high frequency electrode which is held against an intracavitary wall, for example, for cauterization of tissues. The insertion member is in an elongated narrow shape and built of a flexible structure which is flexible in bending directions. In some cases, the insertion member is in the shape of a rigid rod. Normally, an endoscopic high frequency instrument of this sort is introduced into a body cavity by way of a guide means. Typical of such guide means is a biopsy channel which is provided internally of an endoscopic insertion member. In the case of a high frequency instrument having a rigid rod type insertion member, a trocar is often resorted to as a guide means.

As an endoscopic surgical instrument, there have been in use grasping forceps having a pair of grasper members manipulably at a distal end of an insertion member, which is connected to a manipulation means to manipulate opening and closing actions of the grasper members by remote control. For this purpose, it is the general practice to drive the grasper members of the forceps through a link mechanism. For driving the link mechanism, an operating wire is threaded internally of the elongated insertion member to serve as a manipulating force transmitting means, the operating wire being extended as far as a manipulation handle which is attached to the proximal end of the insertion member. An endoscopic forceps of this sort is used, for example, for sampling tissues, for extraction of a diseased part or for other purposes.

There has also been known another type of endoscopic surgical instrument having a forceps manipulably at a distal end of an elongated insertion member, in such a way as to serve as an electrode in a high frequency treatment. In addition to the functions of gripping, sampling and extracting tissues, this forceps-tipped surgical instrument is capable of cauterizating tissues by conduction of a high frequency current through the forceps, broadening the range of its use. For instance, disclosed in Japanese Laid-Open Patent Application H8-509623 (Patent Literature 1) and Japanese Laid-Open Patent Application 2005-261514 (Patent Literature 2) are forceps which are capable of coagulating tissues for hemostatic purposes. Further, disclosed in Japanese Laid-Open Patent Application 2000-70280 (Patent Literature 3) is a forceps type surgical instrument with a function of dissecting diseased tissues in addition to the function of a coagulative hemostatic treatment as mentioned above.

US 2006/264930 A1 discloses a surgical instrument according to the preamble of claim 1.

In Patent Literature 1 mentioned above, the endoscopic forceps is provided with a pair of cup-shaped grasper members which are capable of holding tissues therein when closed. When closed, only marginal edges of arcuately shaped cup portions of the grasper members are brought into abutting engagement with each other. In this case, tissues can be dissected by sharp saw teeth which are formed along the abutting marginal edges through which a high frequency current is conducted. One of the paired grasper members of the forceps is connected to a positive pole while the other one is connected to a negative pole of a high frequency power source, constituting a bipolar electrode across which a high frequency current is conducted at the time of cauterizing tissues.

Further, in Patent Literature 2, a pair of grasper members of a forceps is formed in the shape of a beak which can serve as electrodes. In this case, the grasper members of the forceps are not cup-shaped, and are each in a square waveform on the inner meeting side. Accordingly, a high frequency current is conducted across the paired grasper members of the forceps in a relatively low current density because the inner sides of the graspers are almost entirely held in abutting engagement with tissues which are gripped between the two grasper members. Thus, the forceps performs a function of stanching hemorrhage without dissecting grasped tissues.

Further, in the case of the high frequency surgical instrument disclosed in Patent Literature 3 mentioned above, for the purpose of gripping tissues for a coagulative treatment or for dissection, a pair of saw-toothed grasper members of a forceps is provided at a distal end of an elongated insertion member to be introduced into a body cavity. Similarly to Japanese Laid-Open Patent Application 8-509623 mentioned above, the grasper members of the forceps are arranged as a bipolar electrode across which a high frequency current is conducted at the time of coagulation or dissection of tissues gripped in the forceps. In order to prevent short-circuiting between the grasper members, at least one of the grasper members is electrically insulated on the inner side of a tip end portion, and kept out of contact with the other grasper member except the insulated tip end portion.

As mentioned above, a high frequency surgical instrument tipped with a forceps is capable of performing, in addition to functions as a grasping forceps such as functions of gripping and sampling tissues, a function of a high frequency treatment like cauterization of tissues by conduction of a high frequency current. Of the published Patent Literatures mentioned above, paired grasper members of the forceps in Patent Literatures 1 and 2 have substantially the same outside diameter as an insertion member at a proximal end which is connected to the latter. More specifically, grasper members in Patent Literature 1 are formed in a cup-like shape while grasper members in Patent Literature 2 are narrowed down in a forward direction in the fashion of a beak from a base end which is substantially as thick as an insertion member in outside diameter similarly to the grasper members in Patent Literature 1. Accordingly, a coagulative cauterization treatment is possible with the high frequency instruments described in Patent Literatures 1 and 2 for a hemostatic treatement, but these instruments are not suited for use in a surgery involving a dissection or incision of a mucous membrane or the like. Further, cauterization of an unnecessary part may take place if a high frequency current is conducted through forceps inadvertently while they are still in contact with tissues which do not need cauterization.

The high frequency instrument of Patent Literature 3 is capable of not only a hemostatic treatment by cauterization but also dissection or incision of tissues or mucous membrane by conducting a high frequency while gripping the tissues or mucous membrane between the grasper members. In this regard, in order to dissect tissue quickly in an efficient manner, it is necessary for the grasping fingers to a gripping portion which is narrow enough in width for ensuring a higher current density. In this regard, Patent Literature 3 shows grasper members which are smaller in width as compared with outside diameter of an insertion member.

In Patent Literature 3, an electrically insulated area is provided on a fore end portion of a saw-toothed grasper member, and the two grasper members are spaced apart and intervened by a gap space behind the electrically insulated area in a fore end portion. Thus, a dissectible area is limited by the provision of the electrically insulated area, and, due to the existence of the intervening gap space between the two grasper members, cutting quality as well as efficiency of a dissecting treatment is degraded to a considerable degree. In this regard, it may be conceivable to elongate the grasper members to cope with larger dissections. However, elongation of narrow grasper members will give rise to another problem, that is, a detrimental reduction in physical strength of the grasper members.

### [SUMMARY OF THE INVENTION]

With the foregoing situations in view, it is an object of the present invention to provide an endoscopic high frequency surgical instrument with forceps, which can perform, in addition to basic functions as a grasping forceps, functions of a high frequency surgical instrument such as cauterization and dissection of tissues with a high degree of safety. The invention is defined in claim 1. Further aspects and preferred embodiments are defined in the dependent claims. Aspects, embodiments and examples of the present disclosure which do not fall under the scope of the appended claims do not form part of the invention and are merely provided for illustrative purposes.

In order to achieve the above-mentioned objective, there is provided a high frequency surgical instrument, comprising: a forceps assembly composed of a pair of opening and closing grasper members each having a series of saw tooth portions along an entire width of a grip portion on a meeting inner side in such a way that saw tooth portions on the two grasper member are at least partly brought into abutting engagement against each other when the forceps is closed; an elongated insertion member connected to the forceps assembly at a fore distal end, the insertion member internally providing a passage for a manipulation force transmission means for opening and closing the grasper members of the forceps assembly; and a manipulation handle connected to a proximal end of the insertion member to manipulate opening and closing actions of the grasper members through the manipulation force transmission means; the grasper members being formed of a conducting material in a width smaller than outside diameter of the insertion member and entirely covered with an insulating coat except the saw tooth portions.

The grasper members of the forceps are basically capable of performing the functions of a grasping forceps, e.g., a function of grasping tissues. Meeting inner sides of the respective grasper members are formed with a series of saw tooth portions in such a way that opposing apexes of the saw tooth portions on the two grasper members are brought into abutting engagement against each other when the grasper members are closed, or formed with sat tooth portions with a shift of 1/2 pitch relative to each other so that the saw tooth portions on the two grasper members are wholly brought into abutting engagement in a meshed state when the grasper members are closed. In case the saw tooth portions on the two grasper members are arranged to come into abutting engagement against each other at opposing apex portions, the saw tooth portions are driven to bite on grasped tissues when the grasper members are closed. On the other hand, in case the saw tooth portions on the two grasper members are arranged to come into abutting engagement wholly in a meshed state, it is possible to conduct a high frequency current with uniform current density along the entire length of the grasper members.

Focusing on the function of grasping tissues, it is not important for the grasper members of forceps to have a large width. Namely, the grasping function is not impaired to a detrimental degree as long as the grasper members have a sufficient length. In this regard, narrow grasper members are advantageous in that they have a higher gripping pressure per unit area. However, reduction in width of grasper members can result in a reduction in strength. In this regard, a sufficient strength can be secured as long as the grasper members have a sufficient thickness. From a standpoint of increasing a current density in a cauterization treatment on tissues, it is desirable to employ narrow grasper members which are reduced in width. Of the entire body of the grasper member which is made of a conducting material, it is the inner saw-toothed side that functions as an electrode in a cauterization treatment. Except saw tooth portions on the inner meeting side, each one of the grasper members is entirely covered with an insulating coat by coating with fluorescent resin, ceramic and the like. In order to let the grasper members function as an electrode over the entire length thereof, the saw-toothed grip portions on the inner meeting side are left in an exposed state, free of the insulating coat.

In this manner, the grasper members of a forceps are desirable to be reduced in width on the inner meeting side which plays a role as an electrode at the time of conduction of a high frequency current. Namely, it is only the saw tooth portions on the inner meeting side that need to have a reduced width. Thus, the grasper members may be formed in a uniform width from an inner meeting side to an opposite outer side, or alternatively may be increased in width continuously from an inner meeting side of an opposite outer side for the purpose of enhancing a physical strength. Especially, fortification of fore end portions of grasper members, which are supported in the fashion of a cantilever, will be reflected by stabilized grasping actions and a higher grasping capacity.

The width of each one the grasper members of the forceps is preferably 1/2 or smaller than 1/2 of the outside diameter of the flexible cord of the insertion member, more preferably in the range of 1/2 to 1/6 as compared with the outside diameter of the flexible cord. In this instance, "the width of the grasper member" means the width of the saw-toothed inner side of the grasper member, and "the length of the grasper member" means the length of saw tooth portions in the axial direction of the flexible cord. Further, relative to a thickness in a direction perpendicular to the inner meeting side, each grasper member is formed in a reduced width which is as large as or smaller than 1/2 of the outside diameter of the flexible cord of the insertion member. At least in length and thickness, the grasper members can be dimensioned similarly to conventional grasper forceps. In case the grasper members are increased in width continuously from inner to outer side, each grasper member can have a doubled width at the outer side as compared with its width at the inner side.

The high frequency surgical instrument with forceps functions according to the present invention is provided with an insertion member in the form of a flexible cord or a rigid rod to be introduced into a body cavity through a guide means, e.g., through an endoscopic biopsy channel or a trocar. Any way, the outside diameter of the insertion member should be smaller than the inside diameter of the guide means. In consideration of controllability of the insertion member in a guide means, especially in an endoscopic biopsy channel, at maximum, the flexible cord should have an outside diameter which is slightly smaller than the inside diameter of the endoscopic biopsy channel. More specifically, for example, in a case where the outside diameter of a flexible cord is 1.5mm to 3.0mm, the grasper members are formed in a width of 0.5mm to 1.0mm and in other respects dimensioned similarly to grasper forceps in general. If the width is smaller than 0.5mm, the grasper members will become deficient in the function of grasping tissues or in strength. On the other hand, if greater than 1.0mm, it will become difficult to increase a current density to a sufficient degree in a high frequency treatment.

Using the grasping forceps of the construction as described above, a mucous portion can be pinched and lifted up from an intracavitary wall by the use of the grasper members, and, after a safety check, a high frequency current is conducted therethrough, for example, for a hemostatic treatment or for dissection of a diseased portion. At this time, even if part of the grasper members is in contact with other tissues, there is no possibility of perforating or rupturing a muscular layer or the like. Since exterior surfaces of the grasper members are entirely covered with an insulating coat except the respective saw-toothed inner sides, cauterization is limited to a target portion between the two grasper members at the time of conduction of a high frequency current.

Further, the grasper members of the forceps, which are reduced in width in the fashion of a narrow blade, can be manipulated like scissors for severing a mucous membrane layer or the like. For example, the forceps of the present invention can be advantageously utilized as an instrument for severing a mucous membrane in an endoscopic surgical operation involving the techniques of ESD (Endoscopic Submucosal Dissection). A mucous membrane can be easily dissected by manipulating the forceps to close the grasper members while conducting a high frequency current therethrough. In addition, in each one of the surgical treatments mentioned above, the narrow grasper members of the forceps can be monitored in an endoscopic observation view field and thus can be manipulated in such a way as to hit a target portion with high accuracy.

For manipulating opening and closing actions of the paired grasper members through a link mechanism, the grasper members are connected, for example, to an operating wire serving as a manipulative force transmission means. This operating wire can be utilized as a power supply line instead of providing an independent power supply cable. Further, in a case where the manipulation handle is constituted by a combination of a shaft member and a slider which is fitted slidably on the shaft member, the operating wire is connected to the slider member on which a terminal member is provided for disconnectibly connecting a cable from a high frequency power source.

The above and other objects, features and advantages of the invention will become apparent from the following description of preferred embodiments, taken in conjunction with the accompanying drawings which show by way of example preferred embodiments of the invention. Needless to say, the present invention is not limited to the particular forms shown in the drawings.

### [BRIEF DESCRIPTION OF THE DRAWINGS]

In the accompanying drawings:
Fig. 1 is a schematic view of an endoscopic high frequency surgical instrument with a forceps according to a first embodiment of the invention;
Fig. 2 is a partly sectioned schematic view of a forceps assembly manipulably attached to a fore distal end of a flexible cord of an insertion member;
Fig. 3 is a partly sectioned schematic view similar to Fig. 2, showing grasper members of the forceps in a different position from Fig. 2;
Fig. 4 is a longitudinal sectional view showing the manner in which a proximal end of the flexible cord is connected to a manipulation handle;
Fig. 5 is a plan view of the forceps of Fig. 2;
Fig. 6(a) and 6(b) are sectional views showing dimensional differences between the grasper member in the first embodiment of the invention and a grasper member of alligator forceps in general;
Fig. 7 is a partly cutaway schematic view showing the manner in which a forceps assembly is attached to a flexible cord of an insertion member in a second embodiment of the invention;
Fig. 8 is a partly cutaway schematic view similar to Fig. 7, but showing grasper members of the forceps in a different position from Fig. 7;
Fig. 9 is a plan view of the forceps in Fig. 7; and
Fig. 10 is a front view of the forceps in Fig. 9.

### [BEST MODE FOR CARRYING OUT THE INVENTION]

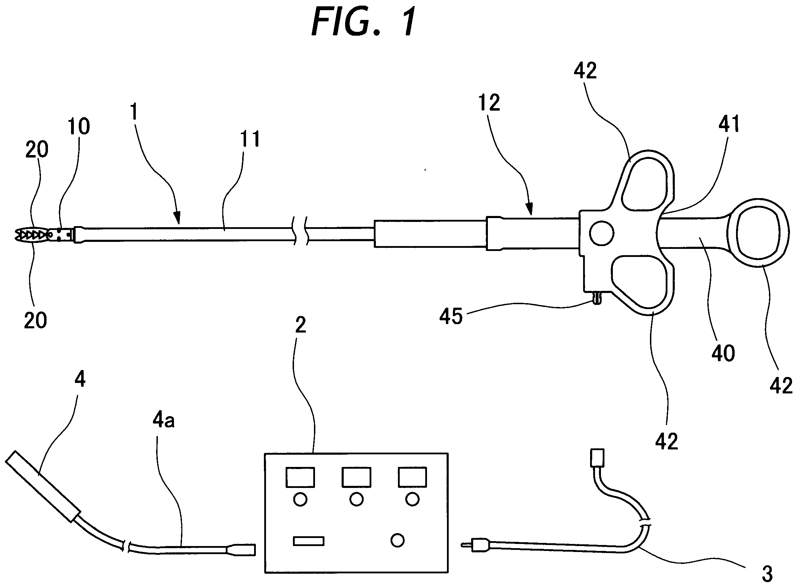

Hereafter, with reference to the accompanying drawings, the invention is described more particularly, initially by way of a first embodiment of the invention. Shown in Fig. 1 is a general layout of a high frequency surgical instrument with forceps according to the present invention. In Fig. 1, indicated at 1 is an endoscopic high frequency surgical instrument tipped with a forceps, and at 2 a high frequency power source. The endoscopic high frequency surgical instrument 1 is a high frequency surgical instrument with functions of a forceps, and connectible to the high frequency power source 2 by way of a cable 3. The high frequency surgical instrument 1 can be introduced into a patient's body cavity as a monopolar type high frequency instrument which is operative in association with a counter electrode in contact with an opposing skin surface of the patient. The endoscopic surgical instrument 1 is composed of a flexible cord 11 serving as an insertion member, a forceps proper 10 which is manipulably attached to a fore distal end of the flexible cord 11, and a manipulation handle 12 attached to a proximal end of the flexible cord 11. The forceps 10 at the fore distal end of the flexible cord 11 is introduced into a body cavity by way of a guide means like an endoscopic biopsy channel.

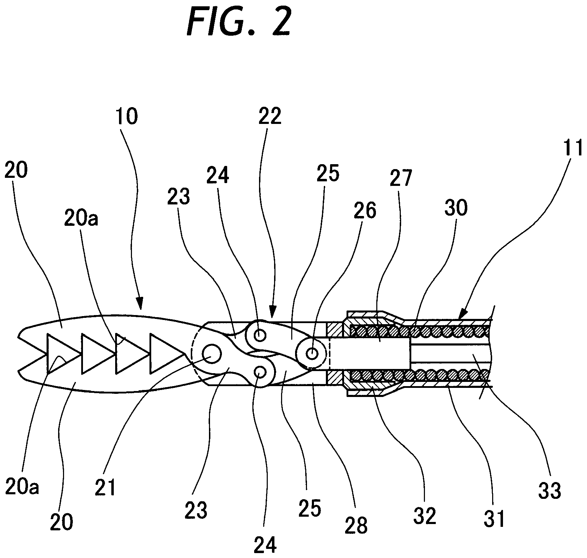

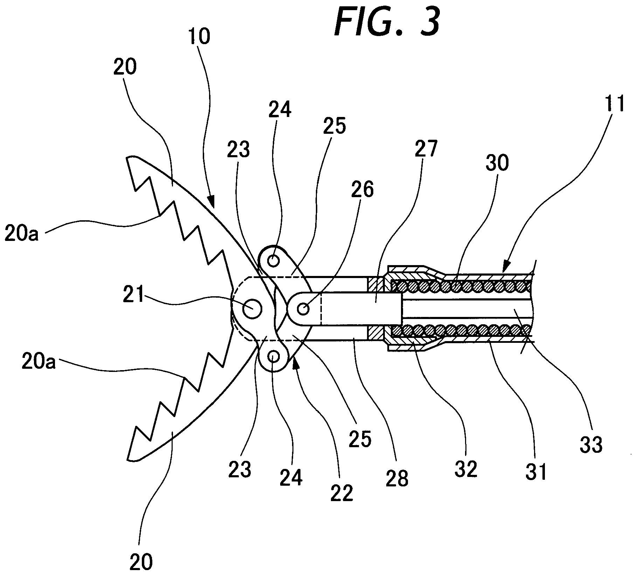

Shown in Figs. 2 and 3 are details in construction of the forceps proper 10, and a manner in which the forceps assembly 10 is attached to the fore end of the flexible cord 11. The forceps proper 10 is provided with paired upper and lower grasper members 20 which are each saw-toothed on the inner meeting side, i.e., shaped with a series of sharply crested saw tooth portions 20a in symmetric relation with a series of saw tooth portions 20a on the other grasper member 20. Each saw tooth portion 20a on the grasper members 20 is shaped by a relatively gentle uphill slope on the front side and an acute downhill slope of an angle of 75 to 90 degrees on the rear side. When the forceps 10 is closed, apexes of the saw tooth portions 20a on one grasper member 20 are brought into abutting engagement with opposing apexes of the saw tooth portions 20a on the other grasper member 20. Thus, the grasper members 20 are capable of gripping tissues to perform functions of grasping forceps which are generally called "alligator forceps." In order to let the grasper members 20 grip tissues in an assured manner, the saw tooth portions 20a are formed across the full width of each grasper member 20.

The two grasper members 20 which constitute the forceps proper 10 are hinged on a pivot pin 21 and can be turned about the pivot pin 21 for movements toward and away from each other. For opening and closing the grasper members 20, for forceps 10 is connected to a link mechanism 22 including front link plates 23 which are pivotally connected to the grasper members 20 on the rear side of the pivot pin 21 and rear link plates 25 which are pivotally connected to the front link plates 23 by pivoting joint pins 24 at the respective fore ends. The other rear ends of the link plates 25 are pivotally connected to a fore end of a drive member 27 by a pivoting joint pin 26. Thus, the grasper members 20 are closed one on the other as shown in Fig. 2 when the drive member 27 is pulled rearward, and the grasper members 20 are spread open as shown in Fig. 3 when the drive member 27 is pushed forward.

The pivot pin 21 is fixedly planted on a mount member 28 which is attached to a fore distal end of the flexible cord 11. The flexible cord 11 is constructed of a tightly wound coil sleeve 30 which is sheathed in an insulating tube 31, and is capable of flexures in bending directions. Fixed on a fore end portion of the flexible tube 30 is a connecting member 32 through which the mount member 28 of the forceps 10 is fixedly attached to the fore distal end of the flexible cord 11. The drive member 27 is extended forward of a fore distal end of the coil sleeve 30 through an opening in the connecting member 32. An operating wire 33 is threaded internally of the coil sleeve 30 and connected to the drive member 27 at its fore distal end.

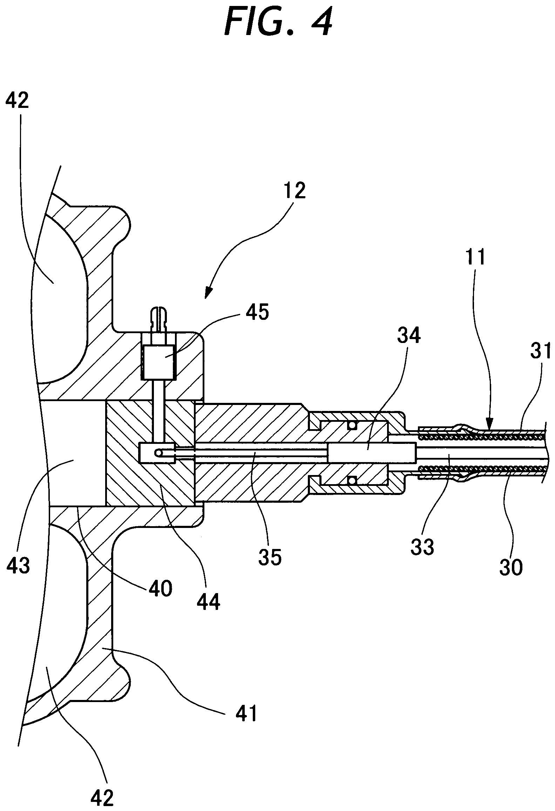

The proximal end of the operating wire 33 is extended into the manipulation handle 12 from a rear end of the flexible cord 11. The manipulation handle 12 is provided with a shaft portion 40 and a slider 41 which is slidably fitted on the shaft portion 40. The handle 12 is manipulable by way of finger rings 42 which are provided at opposite lateral sides of the slider 40 and a finger ring 42 which is provided at a proximal end of the shaft portion 40. As shown in Fig. 4, a slot 43 is formed axially in the shaft portion 40, and a slider block 44 which is fixedly connected with the slider 41 is slidably fitted in the axial slot 43. Further, connected to this slider block 44 is the proximal end of the afore-mentioned operating wire 33. Accordingly, as the slider 41 is pushed or pulled axially along the shaft portion 40, the slider block 44 is moved axially in step with the slider 41 to push or pull the operating wire 33 within the coil sleeve 30. As a consequence, the drive member 27 is moved axially in a forward or backward direction to open or close the grasper members 20 of the forceps 10.

The operating wire 33 is a conducting wire and, if necessary, sheathed in an insulating cover or case. At a proximal end, the operating wire 33 is connected to a sliding member 34, through which a cable 35 is extended out and connected to a terminal member 45 which is provided on the slider block 44. The terminal member 45 on the slider 41 is located in a position which is accessible from outside so that a cable 3 from a high frequency power source 2 can be disconnectibly connected thereto. A cable 4a from the counter electrode 4, to be placed in contact with a patient's skin surface, is also connected to the high frequency power source 2.

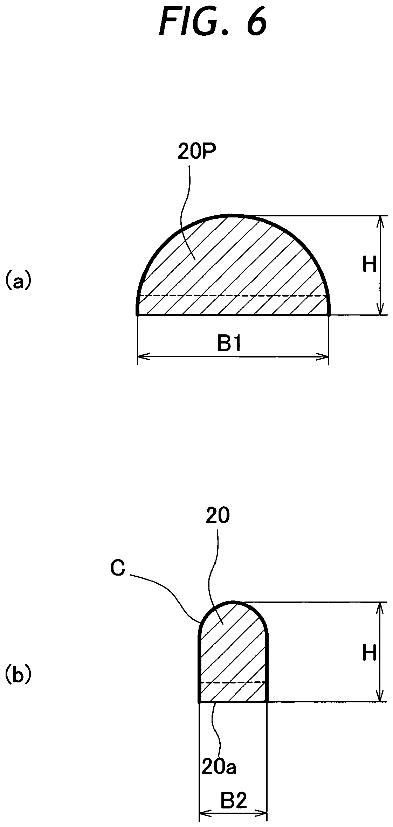

The operating wire 33 is connected to the grasper members 20 of the forceps 10 through the drive member 27, pivot pin 26, link plates 25 and pivoting link pins 24 which are all made of a conducting material. Except contacting surfaces, the drive member 27, pivot pin 26, link plates 25 and pivoting link pins 24 are covered or coated with an insulating material. Accordingly, a power supply path is formed as far as the grasping members 20 of the forceps 10 from the terminal member 45 which is provided on the slider block 44 of the slider 41 on the manipulation handle 12. On the other hand, the grasper members 20 are covered with an insulating coat except the saw tooth portions 20a which are exposed on the meeting inner side of each grasper member 20. This insulation coat of the grasper members 20 is indicated at C in Fig. 6(b). The opposing saw tooth portions 20a of the grasper 20 function as a monopolar electrode in conducting a high frequency current across the forceps 10 and the counter pole 4.

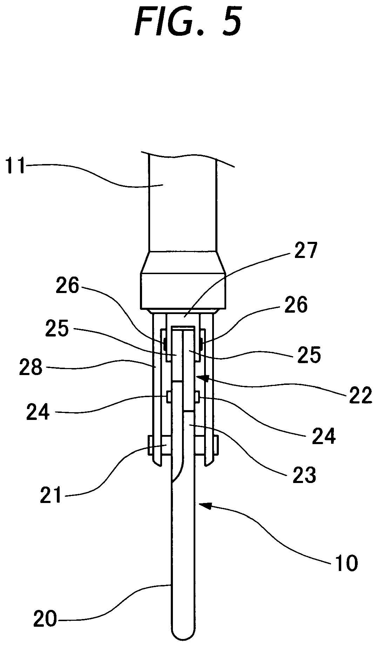

In this instance, in conducting a high frequency current across the forceps 10, forming an electrode on one side, and the counter electrode 4, the current density becomes higher if the grasper members 20 of the forceps 10 are reduced in width. Therefore, as shown in Fig. 5, the forceps 10 employs the grasper members 20 which are narrow and reduced in width. At the time of introduction into a body cavity by way of an endoscopic biopsy channel, the forceps 1 would not obstruct forward movements within the endoscopic biopsy channel as long as its outside diameter is at most equivalent to that of a fore end portion of the flexible cord 11 which is also passed through the endoscopic biopsy channel. However, the grasper members 20 are preferred to be smaller than the flexible cord 11 in outside diameter, more particularly, to be half as thick as the flexible cord 11, e.g., in the range of 0.5mm to 1.0mm in outside diameter. In this regard, the lengths of the grasper members 20 are substantially same as those of grasper members of grasping type forceps in general or shortened to a maximum degree within a range in which stable grasping actions can be ensured. Namely, referring to Figs. 6(a) and 6(b), shown in Fig. 6(a) are dimensions of a grasper member 20P which is adopted by grasping forceps in general. On the other hand, shown in Fig. 6(b) are dimensions of a narrow grasper member 20 which is reduced in width relative to the ordinary grasper member 20P with a width B1. More specifically, relative to the width B1 of the grasper member 20, the narrow grasper member 20 has a width of from 1/2· B1 to 1/4· B1. The narrow grasper member has a maximum height H, which is substantially same as that of the ordinary grasper member 20P. Although not shown in the drawing, the narrow grasper member 20 has a length which is also same as that of the ordinary grasper member 20P.

With a high frequency surgical instrument with a forceps assembly which is arranged in the manner as described above, the forceps 10 is capable of performing functions as a grasping forceps in grasping tissues and at the same time can serve as an electrode in high frequency treatments like cauterization of tissue, making the following techniques feasible.

Firstly, as a grasping instrument, the forceps 10 has a function of grasping tissues. When used only for grasping purposes, the forceps 10 is not connected to the high frequency power source 2. A distal end of the forceps 10 which has been endoscopically inserted into a body cavity is positioned face to face with an organ or tissues to be grasped, and then the slider 41 is pushed or pulled along the shaft portion 40 to open or close the forceps 10. At this time, the grasper members 20 which are reduced in width to approximately 1/2 of the outside diameter of the flexible cord 11 may be considered to have a lowered grasping capacity, but the grasper members 20 of a reduced width can apply a greater gripping pressure and can perform required grasping functions without any problem in particular. The reduction in width is rather advantageous from the standpoint of enhancing fracture strength. Further, the use of the narrow grasper members 20 makes it possible to monitor grasped organ or tissues more clearly through an endoscopic observation system, and to grasp target portions more easily.

Now, when the cable 3 from the high frequency power source 2 is connected to the terminal member 45, a high frequency current can be conducted through the grasper members 20 for a high frequency treatment such as hemostatic cauterization or excision of a diseased portion in a safe and smooth manner. Namely, as soon as a spot of hemorrhage or a diseased portion is spotted by an observation with the endoscopic observation, the forceps 10 is introduced in a guide channel such as a biopsy channel to lead in the vicinity of a portion to be coagulated or removed. The grasper members 20 of the forceps 10 are opened and positioned in front of a target such as a spot of hemorrhage or a diseased portion. Then, the grasper members 20 are closed to grasp the target portion therebetween, and then the forceps 10 is manipulated in such a way as to pull the same into the endoscope. At this time, the forceps 10 is rotated the direction by means of the operation of the flexible cord 11 or the manipulation handle 12 so as to be able to grasp the target portion.

Then, the saw tooth portions 20a on the grasper members 20 are projected obliquely in a rearward direction toward the respective apexes and then turned toward the roots at an acute angle near approximately 75 degrees. When closed, the grasper members 20 are brought into abutting engagement with each other at the apexes of the saw tooth portions 20a. Even if a portion not to be grasped is erroneously captured, there is no problem due to before applying a high frequency current and allow to try again the grasper members 20 to grasp the proper portion. Thus, grasped the correct tissues can be captured securely by the biting actions of the saw tooth portions 20a. As a consequence, a bleeding portion to be coagulated by cauterization or tissues to be dissected can be pulled up from an intracavitary wall, and in this state a high frequency current is applied to the grasper members 20 from the high frequency power source 2. In this instance, since the grasper members 20 are small in width, a high frequency current can be conducted therethrough in a high current density, permitting to carry out a cauterization treatment in an extremely efficient manner.

In this manner, the high frequency forceps is capable of high frequency treatments such as a hemostatic treatment by coagulative cauterization or an excision of a diseased portion. Further, the grasper members 20 are covered with an insulating coat except the saw tooth portions 20a and a cauterizing portion is pulled up from an intracavitary wall, so that there is little possibility of the grasper members 20 cauterizing other portions, or piercing other portions or damaging normal tissues, thus guaranteeing a high degree of safety while permitting to carry out a cauterization treatment or other high frequency treatment smoothly in a facilitated manner.

In addition, a high frequency current is conducted through the entire surfaces of the saw tooth portions 20a of the grasper members 20 holding a grip on tissues. The grasper members 20 are each narrow in width and opened and closed through the link mechanism 22. Therefore, when dissecting or excising a mucous membrane around a diseased portion by ESD techniques, for example, the forceps assembly 10 can be manipulated in the fashion of a pair of scissors in dissecting a mucous membrane and a submucosal layer. More particularly, for this purpose, one of opened grasper members 20 is pierced into the submucosal layer and then manipulated in such a way as to lift up a mucous membrane. The lifted mucous membrane can be dissected easily and safely by operating the grasper members 20 and conducting a high frequency current therethrough. The length of dissection can be extended by advancing the forceps 10 while repeating opening and closing actions of the grasper members 20, and then conducting a high frequency current. Thereby, one can be precisely conducted dissecting or excising a mucous membrane around a diseased portion. Even if the grasper member 20 in the submucosal layer happens to come into contact with an underlying muscular layer, there is no possibility of cauterizing or damaging the muscular layer because contacting portions of the grasper member 20 are covered with an insulating coat.

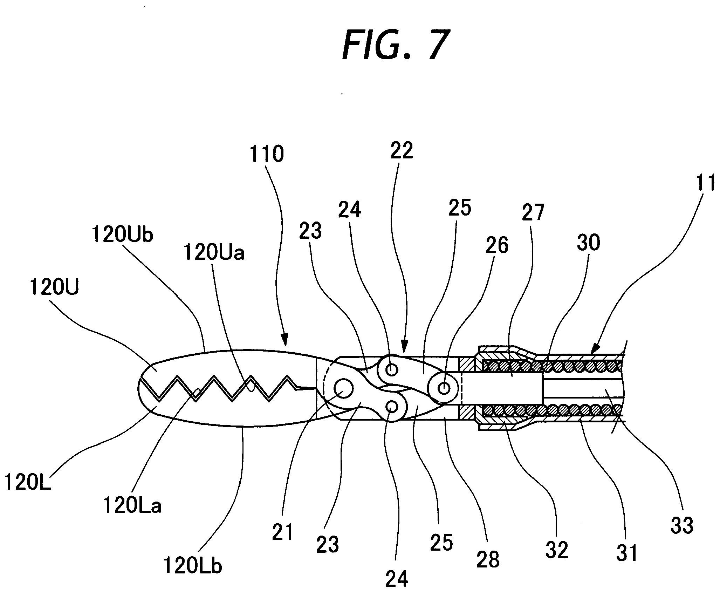

Shown in Figs. 7 through 10 is a second embodiment of the present invention. Similarly to the counterpart in the foregoing first embodiment, a forceps proper 110 in the second embodiment is connectible to the high frequency power source 2 of Fig. 1 by way of a cable 3. Except for a modification in construction of grasper members 120, the forceps 110 of the second embodiment is constructed in the same way as the forceps 10 of the first embodiment. Therefore, in the following description of the second embodiment, the component parts which are equivalent with a counterpart in the first embodiment are simply designated by the same reference numeral or character, without repeating similar descriptions.

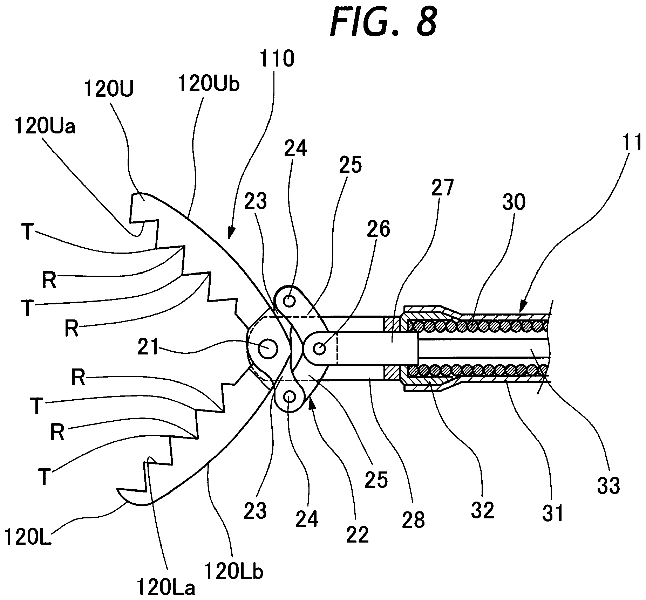

In contrast to the grasper members 20 of the first embodiment which are toothed symmetrically relative to each other as shown in Figs. 2 and 3, the grasper members 120U and 120L of the second embodiment are provided with a series of saw tooth portions 120Ua or 120La as shown in Figs. 7 and 8, apexes and roots T and R (Fig. 8) of saw tooth portions 120Ua or 120La on one grasper member 120U or 120L are shifted by a 1/2 pitch in the axial direction relative to saw tooth portions 120La or 120Ua on the other grasper member 120L or 120U. Thus, when the grasper members 120U and 120L are closed, the apexes and roots T and R of the saw tooth portions 120Ua or 120La on one grasper member 120U or 120L are brought into abutting engagement with roots and apexes T and R of saw tooth portions 120La or 120Ua on the other grasper member 120L or 120U in a meshed state as shown in Fig. 7. That is to say, when closed, the saw tooth portions 120Ua and 120La on the grasper members 120U and 120L come into abutting engagement at and along the entire sloped surfaces of the saw tooth portions 120Ua and 120La. Namely, despite the existence of apexes T and roots R, the inner meeting sides of the grasper members 120U and 120L are disposed substantially at a uniform distance from each other at all positions in the longitudinal direction.

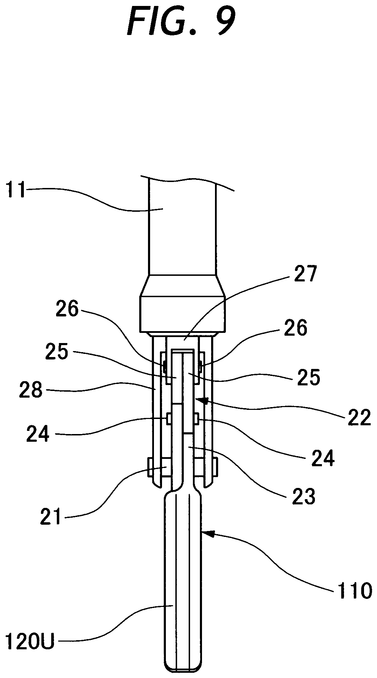

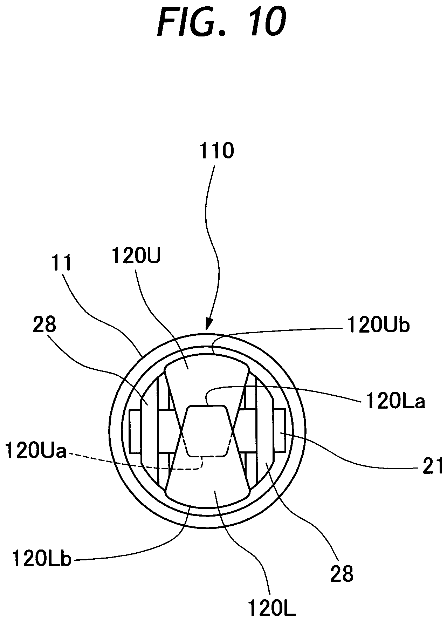

Further, as shown in Figs. 9 and 10, the grasper members 120U and 120L are continuously increased in width from the inner side with the saw tooth portions 120Ua or 120La toward an opposite outer side 120Ub or 120Lb, i.e., toward the side away from the saw tooth portions 120Ua or 120La. In other words, the grasper members 120U and 120L have tapered lateral sides which are diverged in an outward direction. In this regard, it is desirable for the grasper members 120U and 120L to have approximately a double width at the outer side 120Ub or 120Lb as compared with a width on the inner side shaped with the saw tooth portions 120Ua or 120La. Similarly to the counterparts in the foregoing embodiment, except the saw tooth portions 120Ua and 120La, the grasper members 120U and 120L are entirely covered with an insulating coat including surfaces at the outer side 120Ub or 120Lb.

By arranging the forceps 110 in the manner as described above, when the grasper members 120U and 120L are manipulated to grasp bleeding tissues therebetween for hemostatic purposes, the opposing saw tooth portions 120Ua and 120La on the grasper members 120U and 120L are uniformly spaced apart at both apexes and roots of the saw tooth portions 120Ua and 120La, applying uniform pressures on grasped tissues to effect a uniform coagulative cauterization treatment feasible over a broad range. Besides, tissues can be dissected in an efficient manner by the opposing saw tooth portions 120Ua and 120La which are brought into abutting engagement against each other upon closing the grasper members 120U and 120L.

Furthermore, the grasper members 120U and 120L, which are pivotally supported on the pivot pin 21 at a base end in a cantilever fashion, are increased in width or in wall thickness toward the respective outer sides 120Ub and 120Lb and thus are imparted with an enhanced strength. Accordingly, the grasper members 120U and 120L can apply a strong grasping force at the time of grasping tissues without undergoing deformations, ensuring high operational stability along with a high grasping capacity for tissues or other parts. Since the grasper members 120U and 120L are covered with an insulating coat except the saw tooth portions 120Ua and 120La, there is no possibility of cauterizing tissues in contact with the lateral sides of the grasper members 120U and 120L, which are diverged toward the outer sides 120Ub and 120Lb, respectively.

## Claims

1. A high frequency surgical instrument having a forceps assembly (10, 110) composed of a pair of opening and closing grasper members (20, 120U, 120L) supported by a pivot pin (21) in a cantilever fashion, each of said grasper members (20, 120U, 120L) having a series of saw tooth portions (20a, 120Ua, 120La) formed along an entire width of a grip portion on a meeting inner surface from a distal end and along an entire length thereof, the inner surfaces being at least partly brought into contact with each other when said forceps assembly (10, 110) is closed, an elongated insertion member (11) connected to said forceps assembly (10, 110) at a fore distal end, said insertion member (11) internally providing a passage for a manipulation force transmission means (33) connected to a high frequency power source (2) and adapted for opening and closing said grasper members (20, 120U, 120L) of said forceps assembly (10, 110), and a manipulation handle (12) connected to a proximal end of said insertion member (11) to manipulate opening and closing actions of said grasper members (20, 120U, 120L) through said manipulation force transmission means (33), **characterised in that**:
said grasper members (120U, 120L) are formed of a conducting material in a width smaller than outside diameter of said insertion member (11);
said saw tooth portions (20a, 120Ua, 120La) of said grasper members (120U, 120L) are electrically connected to said high frequency power source (2);
exposed surface of said grasper members (120U, 120L) are covered with an insulating coat except the inner meeting surface of said saw tooth portions (120Ua, 120La) formed along the entire length of the inner surfaces;
side surfaces of each of said grasper members (120U, 120L) are tapered such that the width is continuously increased from said inner surface to an outer surface opposite to said surface.

2. A high frequency surgical instrument as set forth in claim 1, wherein said grasper members (120U, 120L) are formed with said saw tooth portions (120Ua, 120La) symmetrically relative to each other such that apexes of saw tooth portions on one grasper member are brought into abutting engagement with apexes of saw tooth portion of the other grasper member when said forceps is closed.

3. A high frequency surgical instrument as set forth in claim 1, wherein saw tooth portions (20a, 120Ua, 120La) on one grasper member are shifted by a half pitch relative to saw tooth portions on the other grasper member such that saw tooth portions on the two grasper members are brought into meshed engagement and entirely abutted against each other when said forceps is closed.

4. A high frequency surgical instrument as set forth in claim 1, wherein said grasper members are formed in a width as small as or smaller than 1/2 of outside diameter of said insertion member.

5. A high frequency surgical instrument as set forth in claim 1, wherein said insertion member is in the form of a flexible cord having an operating wire of said manipulation force transmitting means passed through a flexible sleeve.

## Patentansprüche

1. Chirurgisches Hochfrequenzinstrument mit einer Zangenanordnung (10, 110), bestehend aus einem Paar zu öffnender und zu schließender Greifglieder (20, 120U, 120L), die durch einen Schwenkzapfen (21) nach Freiträgerart gelagert sind, wobei jedes der Greifglieder (20, 120U, 120L) eine Reihe von Sägezahnabschnitten (20a, 120Ua, 120La) aufweist, gebildet entlang einer gesamten Breite eines Griffabschnitts an einer inneren Passfläche von einem distalen Ende und entlang ihrer gesamten Länge, wobei die Innenflächen zumindest teilweise in Berührung miteinander gebracht werden, wenn die Zangenanordnung (10, 110) geschlossen wird, einem langgestreckten Einführelement (11), das an der Zangenanordnung (10, 110) an einem vorderen distalen Ende angebracht ist, wobei das Einführelement (11) innen mit einem Kanal für eine Manipulierkraft-Übertragungseinrichtung (33) versehen ist, welche mit einer Hochfrequenz-Leistungsquelle (2) verbunden und dazu ausgebildet ist, die Greifglieder (20, 120U, 120L) der Zangenanordnung (10, 110) zu öffnen und zu schließen, und einem Manipulierhandgriff (12), der an ein proximales Ende des Einführelements (11) angeschlossen ist, um Öffnungs- und Schließvorgänge der Greifglieder (20, 120U, 120L) über die Manipulierkraft-Übertragungseinrichtung (33) zu manipulieren,
**dadurch gekennzeichnet, dass**
die Greifglieder (120U, 120L) aus einem leitenden Werkstoff mit einer Breite geringer als der Außendurchmesser des Einführelements (11) gebildet sind;
die Sägezahnabschnitte (20a, 120Ua, 120La) der Greifglieder (120U, 120L) elektrisch mit der Hochfrequenz-Leistungsquelle (2) verbunden sind;
die freiliegende Oberfläche der Greifglieder (120U, 120L) mit einem Isolierüberzug bedeckt sind, ausgenommen die innere Passfläche der Sägezahnabschnitte (120Ua, 120La), ausgebildet entlang der gesamten Länge der Innenfläche; und
Seitenflächen jedes der Greifglieder (120U, 120L) derart verjüngt ausgebildet sind, dass die Breite kontinuierlich zunimmt von der Innenfläche zu einer Außenfläche abgewandt von der Oberfläche.

2. Chirurgisches Hochfrequenzinstrument nach Anspruch 1, bei dem die Greifglieder (120U, 120L) mit den Sägezahnabschnitten (120Ua, 120La) symmetrisch in Bezug aufeinander derart ausgebildet sind, dass die Scheitel der Sägezahnabschnitte an einem Greifglied in Anlage gebracht werden mit Scheiteln des Sägezahnabschnitts des anderen Greifglieds, wenn die Zange geschlossen ist.

3. Chirurgisches Hochfrequenzinstrument nach Anspruch 1, bei dem die Sägezahnabschnitte (20a, 120Ua, 120La) an einem Greifglied um einen halben Mittenabstand in Bezug auf Sägezahnabschnitte des anderen Greifglieds versetzt sind, so dass Sägezahnabschnitte der beiden Greifglieder in kämmenden Eingriff miteinander gebracht werden und vollständig aneinander anliegen, wenn die Zange geschlossen ist.

4. Chirurgisches Hochfrequenzinstrument nach Anspruch 1, bei dem die Greifglieder in einer Breite von oder kleiner als 1/2 des Außendurchmessers des Einführelements ausgebildet sind.

5. Chirurgisches Hochfrequenzinstrument nach Anspruch 1, bei dem das Einführelement die Form einer flexiblen Schnur mit einem Betätigungsdraht der Manipulierkraft-Übertragungseinrichtung aufweist, die durch eine flexible Hülse geführt ist.

## Revendications

1. Instrument chirurgical à hautes fréquences présentant un ensemble de pince (10, 110) composé d'une paire d'éléments de préhension d'ouverture et de fermeture (20, 120U, 120L) supporté par une broche d'articulation (21) d'une manière en porte-à-faux, chacun desdits éléments de préhension (20, 120U, 120L) présentant une série de portions en dents de scie (20a, 120Ua, 120La) formées sur toute une longueur d'une portion de serrage sur une surface intérieure de réunion à partir d'une extrémité distale et sur toute une longueur de ceux-ci, les surfaces intérieures étant au moins en partie amenées en contact l'une avec l'autre lorsque ledit ensemble de pince (10, 110) est fermé, un élément d'introduction allongé (11) relié audit ensemble de pince (10, 110) sur une extrémité distale avant, ledit élément d'introduction (11) fournissant en interne un passage pour un moyen de transmission de force de manipulation (33) relié à une source d'alimentation de hautes fréquences (2) et apte à ouvrir et fermer lesdits éléments de préhension (20, 120U, 120L) dudit ensemble de pince (10, 110), et une poignée de manipulation (12) reliée à une extrémité proximale dudit élément d'introduction (11) afin de manipuler des actions d'ouverture et de fermeture desdits éléments de préhension (20, 120U, 120L) par le biais dudit moyen de transmission de force de manipulation (33),
**caractérisé en ce que**
lesdits éléments de préhension (120U, 120L) sont formés d'un matériau conducteur d'une largeur inférieure au diamètre extérieur dudit élément d'introduction (11) ;
lesdites portions en dents de scie (20a, 120Ua, 120La) desdits éléments de préhension (120U, 120L) sont reliées par voie électrique à ladite source d'alimentation de hautes fréquences (2) ; des surfaces exposées desdits éléments de préhension (120U, 120L) sont recouvertes d'un revêtement isolant, à l'exception de la surface de réunion intérieure desdits portions en dents de scie (120Ua, 120La) formés le long de la longueur tout entière des surfaces intérieures, et des surfaces latérales de chacun desdits éléments de préhension (120U, 120L) présentent une diminution progressive, de sorte que la largeur est augmentée en continu de ladite surface intérieure à une surface extérieure opposée à ladite surface.

2. Instrument chirurgical à hautes fréquences selon la revendication 1, dans lequel lesdits éléments de préhension (120U, 120L) sont formés avec lesdites portions en dents de scie (120Ua, 120La) de manière symétrique entre eux, de sorte que des apex de portions en dents de scie sur un élément de préhension sont amenés en contact d'aboutement avec des apex de partie en dents de scie de l'autre élément de préhension lorsque ladite pince est fermée.

3. Instrument chirurgical à hautes fréquences selon la revendication 1, dans lequel lesdites portions en dents de scie (20a, 120Ua, 120La) sur un élément de préhension sont déplacées d'un demi-pas par rapport à des portions en dents de scie sur l'autre élément de préhension, de sorte que les portions en dents de scie sur les deux éléments de préhension sont amenées à former une prise par engrènement et sont entièrement agencées bout à bout l'une contre l'autre lorsque ladite pince est fermée.

4. Instrument chirurgical à hautes fréquences selon la revendication 1, dans lequel lesdits éléments de préhension sont formés dans une largeur aussi petite ou plus petite que ½ du diamètre extérieur dudit élément d'introduction.

5. Instrument chirurgical à hautes fréquences selon la revendication 1, dans lequel ledit élément d'introduction se présente sous le forme d'un cordon flexible présentant un fil de manoeuvre dudit moyen de transmission de force de transmission passé à travers un manchon flexible.
